# EUROPEAN PATENT APPLICATION

(11) **EP 4 481 035 A1**
(43) Date of publication of application: **25.12.2024**
(21) Application number: 23755827.5
(22) Date of filing: 16.02.2023
(51) Int. Cl.: C12N 5/0797, C07K 14/705, A61K 35/30

(54) **HUMAN-INDUCED PLURIPOTENT STEM CELL OVEREXPRESSING TLX AND USE THEREOF**

(30) Priority: 18.02.2022 CN 202210151789
(71) Applicant: Panexo Biotech Sg Pte.Ltd, Singapore 409051 (SG)
(72) Inventor: WANG, Yi, Beijing 100141 (CN); SONG, Haifeng, Beijing 100141 (CN); XU, Mingzhi, Beijing 100141 (CN); CHEN, Gang, Beijing 100141 (CN); DONG, Yanan, Beijing 100141 (CN); XUE, Miaomiao, Beijing 100141 (CN); WANG, Danfeng, Beijing 100141 (CN)
(74) Representative: Michalski Hüttermann & Partner Patentanwälte mbB
(86) International application number: PCT/CN2023/076366
(87) International publication number: WO 2023/155825

(57) **Abstract**

Provided are a human-induced pluripotent stem cell overexpressing TLX and use thereof. By enabling a human-induced pluripotent stem cell (hiPSC) to overexpress TLX or a truncation thereof, the self-driven differentiation of hiPSC into NSC is achieved.. By employing this solution, not only the differentiation of hiPSC into NSC is accelerated, but also long-term stable in-vitro passaging of NSC is achieved, so that donor cells are provided for the treatment of nervous system diseases, cell therapy, and the like, and the obtained NSC exosome has good biological activity.

## Description

This application claims the priority of Chinese Patent Application No. 202210151789.5, filed with the China National Intellectual Property Administration on February 18, 2022, and titled with "HUMAN-INDUCED PLURIPOTENT STEM CELL OVEREXPRESSING TLX AND USE THEREOF", the disclosure of which is hereby incorporated by reference in its entirety.

### FIELD

The present disclosure relates to the technical field of stem cell culture, and in particular to human induced pluripotent stem cell overexpressing TLX and use thereof.

### BACKGROUND

Neural stem cells (NSCs) are pluripotent stem cells capable of unlimited self-renewal and differentiation into neurons, astrocytes, and oligodendrocytes, providing a new option for studying neurogenesis and neurodevelopment, establishing models for neurological diseases and designing systems for drug screening. Inducing differentiation of pluripotent stem cells into NSCs in vitro is the most suitable and convenient means of obtaining NSCs.

Human induced pluripotent stem cells (hiPSCs) and embryonic stem cells (ESCs) are two commonly used pluripotent stem cells. ESCs are derived from human embryonic cells, which are difficult to obtain for ethical reasons. hiPSCs are derived from somatic cells by reprogramming to express OCT3/4, SOX2, KLF4, and c-Myc. hiPSCs possess similar characteristics to embryonic stem cells, making hiPSCs an alternative cell source to ESCs. The differentiation of induced neural stem cells (iNSCs) from hiPSCs has been extensively studied. However, the instability of hiPSC-derived iNSCs in in vitro culture, their inability to proliferate, and their susceptibility to differentiation have hindered the use of iNSCs in research and clinical settings. Therefore, there is an urgent need for a stable passaging culture of hiPSC-induced iNSCs in vitro. In recent years, the long-term stable culture of primary NSCs in vitro has also been extensively studied. Commonly methods used to immortalize primary NSCs include somatic cell fusion, overexpression of c-Myc, V-Myc, SV40 large T antigen, or modification of telomerase. However, these methods can cause NSCs to lose their phenotype or become oncogenic.

In recent years, much attention has been given to intracellular factors (TLX, Sox2, Hes, histone-modifying enzymes, chromatin-remodeling proteins, and small RNA regulators) and extracellular signaling molecules (such as Wnt, Notch, Shh, TGFα, EGF and FGF), which regulate stem cell self-renewal. TLX (also known as NR2E1) is an orphan nuclear receptor that is highly expressed in the mature brain, particularly in NSCs in the subventricular zone (SVZ). It is essential for NSC self-renewal, proliferation and neurogenesis. TLX maintains NSC self-renewal by inhibiting proteins such as Pten/LSDI and p21, and maintains NSC proliferation by regulating the expression of SOX2. In addition, TLX can regulate neural development by regulating Wnt7a, Mmp2, and SIRT1. Using the hippocampus infected with TLX-expressing lentivirus, Murai et al. demonstrated that the hippocampus specifically expressing TLX increased the proliferation of NSCs in a self-driven manner, promoted hippocampal neurogenesis, and repaired the brain's learning and memory functions. TLX overexpression has been reported to be associated with gliomas. However, Li et al. proved that the overexpression of TLX can reduce the proliferation and migration ability of neuroglial stem cells, thereby inhibiting tumorigenesis.

Overexpression of TLX in NSCs can lead to NSCs with self-renewal capability. Current methods for differentiating NSCs from hiPSCs have a long differentiation cycle, a low positive rate of NSCs, and the reagents used have clinical safety concerns. In addition, hiPSC-derived NSCs cannot be stably subcultured for extended periods in vitro, and the reproducibility of NSCs obtained through differentiation between batches is low. This results in an insufficient supply of high-purity NSCs for stable transfection and expression of the TLX gene. Other common methods for transforming primary NSCs into immortalized NSCs include somatic cell fusion, overexpression of c-Myc/V-Myc or SV40 large T antigen, or modification of telomerase. These methods may cause NSCs to lose their phenotype or become oncogenic. In addition, there are ethical controversies surrounding donors of primary NSCs. As a result, it is not yet possible to obtain stable NSCs between batches in a scale-up culture. These problems limit cell therapy for neurological diseases and result in the unavailability of large quantities of high-quality

NSC-derived exosomes.

### SUMMARY

In view of this, the technical problem to be solved by the present disclosure is to provide human induced pluripotent stem cells overexpressing TLX and use thereof in differentiation into immortalized NSC cells.

The present disclosure provides use of TLX in the construction of human induced pluripotent stem cells (hiPSCs) that self-drivingly differentiate into neural stem cells (NSCs), wherein the TLX comprises at least one selected from the group consisting of the following I to IV:
I), a TLX protein having an amino acid sequence set forth in SEQ ID NO: 1,
II), a protein having an amino acid sequence of the TLX protein in I) by substitution, deletion or addition of one or more amino acids and with a function identical or similar to the TLX protein,
III), a nucleic acid molecule encoding the protein in I) or II), and
IV), a nucleic acid molecule having a nucleotide sequence of the nucleic acid molecule in III) by substitution, deletion or addition of one or more nucleotides and encoding a protein with an identical or similar function; and
V), an substance regulating a level or activity of at least one selected from the group consisting of I) to V).

The present disclosure also provides a TLX protein having an amino acid sequence set forth in SEQ ID NO: 1.

Alternatively, the present disclosure provides a truncating variant of a TLX protein. The truncating variant of the present disclosure has an amino acid sequence set forth in SEQ ID NO: 1 by deletion of 1-200 amino acids at N-terminus.

In some embodiments, the truncating variant of a TLX protein has an amino acid sequence set forth in SEQ ID NO: 2.

The present disclosure also provides a nucleic acid encoding a TLX protein. Alternatively, the present disclosure provides a nucleic acid encoding the truncating variant.

In an embodiment of the present disclosure, the nucleic acid has an nucleic acid sequence set forth in SEQ ID NO: 3 and encodes a protein set forth in SEQ ID NO: 1; the nucleic acid has an nucleic acid sequence set forth in SEQ ID NO: 4 and encodes a protein set forth in SEQ ID NO: 2.

The present disclosure also provides a transcription unit comprising a promoter and the nucleic acid of the present disclosure.

In the present disclosure, the promoter is a CMV promoter or an EF1A promoter.

The present disclosure also provides a plasmid vector comprising a backbone vector and the transcription unit of the present disclosure.

The plasmid vector of the present disclosure further comprises a nucleic acid encoding EGFP.

EGFP is used as a reporter gene to facilitate the observation of culture or differentiation effects. Its expression has no effect on cell growth, TLX expression and function.

In the present disclosure, the backbone vector is not limited. Experiments have demonstrated that plasmid vectors commonly used for iPSCs in the art, including a variety of commonly used commercial lentiviral and adenoviral vectors, or stably transfected non-viral expression vectors containing integrase, can be used for the expression of the nucleic acid sequence of the present disclosure.

The present disclosure also provides a host cell transfected or transformed with the plasmid vector of the present disclosure.

The host cell of the present disclosure is a *Escherichia coli* cell, human renal epithelial cell, insect cell or hiPSC.

The present disclosure also provides a human induced pluripotent stem cell (hiPSC) that self-drivingly differentiates into a neural stem cell (NSC), which expresses the nucleic acid of the present disclosure. In the hiPSC, a nucleic acid encoding the protein set forth in SEQ ID NO: 1 is expressed or a nucleic acid encoding the truncating variant set forth in SEQ ID NO: 2 is expressed.

A method for constructing the hiPSC that self-drivingly differentiates into NSC of the present disclosure comprises packaging the plasmid vector and a helper plasmid in a virus to obtain a lentivirus, and infecting hiPSCs to obtain the hiPSCs that self-drivingly differentiate into NSCs.

The present disclosure also provides an immortalized neural stem cell (NSC), obtained by differentiation of the hiPSC of the present disclosure.

The present disclosure also provides a method for differentiating human induced pluripotent stem cells (hiPSCs) into immortalized neural stem cells (NSCs), comprising culturing the hiPSC that self-drivingly differentiates into NSC of the present disclosure to obtain an immortalized NSC.

In the present disclosure, a culture medium for promoting differentiation of hiPSCs into immortalized NSCs comprises a basal culture medium and one selected from the group consisting of BSA, Glutamax additive, sodium pyruvate, NaCl, N2 trophic factor, B27 neurotrophic factor, insulin, nonessential amino acid, FGF2, EGF, heparin and a combination thereof. In an embodiment of the present disclosure, the basal culture medium is Neurobasal medium.

In some embodiments, the culture medium for promoting differentiation of hiPSCs into immortalized NSCs comprises Neurobasal medium, 1 g/100mL -3 g/100mL BSA, 0.5 vol%-1.5 vol% Glutamax additive, 0.5 vol%-1.5 vol% sodium pyruvate, 0.01-0.1 mol/L NaCl, 2 vol%-8 vol% N2 trophic factor, 8 vol%-12 vol% B27 neurotrophic factor, 1×penicillin-streptomycin, 8-12 ng/mL FGF2, and 8-12 ng/mL insulin.

In some preferred embodiments, , the culture medium for promoting differentiation of hiPSCs into immortalized NSCs comprises Neurobasal medium and 2 g/100mL BSA, 1 vol% Glutamax additive, 1 vol% sodium pyruvate, 0.05 mol/L NaCl, 5 vol% N2 trophic factor, 10 vol% B27 neurotrophic factor, 1×penicillin-streptomycin, 10 ng/mL FGF2, and 10 ng/mL insulin.

The present disclosure also provides a method for subculturing the immortalized NSC, wherein the culture medium used for subculturing is a culture medium comprising GSK3 and TGF-beta inhibitors. In an embodiment of the present disclosure, the culture medium comprising GSK3 and TGF-beta inhibitors is TFM medium.

The present disclosure also provides a method for producing an exosome of neural stem cells (NSCs), comprising culturing the immortalized NSC, collecting a supernatant, and extracting an exosome of NSCs.

The method for extraction comprises primary purifying the culture supernatant of the immortalized NSCs by tangential flow ultrafiltration, followed by refined purifying the primary purified sample using Capto Core 700 column (referred to as Core700).

The present disclosure also provides an exosome of neural stem cells (NSCs) produced by the method.

The present disclosure also provides use of the exosome of neural stem cells (NSCs) produced by the method in the manufacture of a medicament for protecting a neuron.

The present disclosure also provides a medicament for use in protecting a neuron, wherein the exosome of neural stem cells (NSCs) produced by the method is used as a raw material.

The present disclosure also provides a method for protecting a neuron, comprising administering the medicament of the present disclosure.

The present disclosure achieves self-driven differentiation of hiPSCs into NSCs by overexpressing TLX or a truncating variant thereof in human induced pluripotent stem cells (hiPSCs), which not only accelerates the differentiation of hiPSCs into NSCs, but also achieves a long-term stable in vitro subculture of NSCs, showing good stemness at least up to 45 passages. This provides donor cells for obtaining NSC exosomes, and the obtained NSC-derived exosomes have good biological activity after purification.

### BRIEF DESCRIPTION OF DRAWINGS

FIG. 1a shows the structure of the plasmid lenti-SFH-EGFP-CMV-NR2E1, FIG. 1b shows the structure of the plasmid lenti-SFH-EGFP-CMV-NR2E1 (182-386aa), FIG. 1c shows the structure of the plasmid lenti-CMV-NR2E1; FIG. 1d shows the structure of the plasmid lenti-CMV-NR2E1 (182-386aa); FIG. 1e shows the structure of the plasmid lenti-Ub-EGFP-EF1a-NR2E1; FIG. 1f shows the structure of the plasmid lenti-Ub-EGFP-EF1a-NR2E1 (182-386aa).
FIG. 2a shows the protein marker; FIG. 2b shows the results of Western Blot.
FIG. 3a shows the morphology of hiPSC^{CMV-FL-TLX} cells; FIG. 3b shows the morphology of hiPSC^{EF1A-FL-TLX} cells; FIG. 3c shows the mRNA expression level of NR2E1 in cells transfected under two promoters separately; FIG. 3d shows the growth curves of cells transfected under two promoters separately; FIG. 3e shows the results of full-length TLX expression in cells transfected under two promoters separately by flow cytometry; FIG. 3f shows the results of EGFP expression in hiPSC^{TLX-FL} cells by flow cytometry.
FIG. 4a shows the morphology of hiPSC^{CMV-TP-TLX} cells; FIG. 4b shows the morphology of hiPSC^{EF1A-TP-TLX} cells; FIG. 4c shows the mRNA expression level of NR2E1 in cells transfected under two promoters separately; FIG. 4d shows the growth curves of cells transfected under two promoters separately; FIG. 4e shows the results of truncated TLX in cells transfected under two promoters separately by flow cytometry; FIG. 4f shows the results of EGFP expression in hiPSC^{TLX-TP} cells by flow cytometry.
FIG. 5 shows a comparison of the conventional flow chart for differentiation into NSCs and the flow chart of the present disclosure for differentiation into NSCs.
FIG. 6A shows the differentiation of iPSC^{TLX-FL} into iNSC^{TLX-FL} induced using different culture media, and there are three images for each medium including brightfield, green fluorescence, and overlay images from left to right, where the characteristic morphology of NSCs is the growth of flower clusters.
FIG. 6B shows the differentiation of iPSC^{TLX-TP} into iNSC^{TLX-TP} induced using different culture media, and there are three images for each medium including brightfield, green fluorescence, and overlay images from left to right, where the characteristic morphology of NSCs is the growth of flower clusters.
FIG. 6C shows the differentiation of iPSC^{TLX-FL/TP} without expressing EGFP into iNSC^{TLX-FL/TP} induced using NGDI medium, where the characteristic morphology of NSCs is the growth of Rosette.
FIG. 7 shows the results of the expression of NSC protein markers Sox2, Nestin, Vimentin, and Musashi in iPSC^{TLX-FL/TP} after cultured in NGDI medium for 3 days by flow cytometry.
FIG. 8 shows the results of the differentiation of wild-type hiPSCs into NSCs after 21 days using a conventional differentiation method by flow cytometry, and the results of the differentiation of hiPSCs overexpressing TLX into NSCs after 6 days of culture in NGDI medium by flow cytometry.
FIG. 9 shows the morphology of iNSC^{TLX} cells cultured in different maintenance media.
FIG. 10 shows the growth curve of iNSC^{TLX-FL/TP} cells.
FIG. 11 shows the results of detecting Flag tags in NSC cells by Western Blot, where NSC^{FL-lenti} and NSC^{TP-lenti} indicate the cells transfected with the viruses lenti-Ub-EGFP-EF1a-NR2E1 and lenti-Ub-EGFP-EF1a-NR2E1 (182-386aa) viral cells, respectively, and NSC^{TP-lenti-EF1a} represents the NSC^{TP-lenti} cells further transfected with the virus lenti-Ub-EGFP-EF1a-NR2E1 (182-386aa).
FIG. 12 shows the morphology of NSCs differentiated from wild-type hiPSCs at passage 1 (P1) and 2 (P2) and the results of expression levels of SOX2/Nestin by flow cytometry; and the morphology of NSCs differentiated from iNSC^{TLX-TP} at passage 1 (P1) and 15 (P15) and the results of expression levels of SOX2/Nestin by flow cytometry.
FIG. 13 shows the expression levels of SOX2, Nestin, Vimentin, and PAX6 in iNSC^{TLX-FL/TP} cells at different passages detected by flow cytometry.
FIG. 14 shows the expression levels of Vimentin, Tuj 1, Pax6, and Nestin in NSC^{TP-TLX} cells at passage 15 detected by immunofluorescence.
FIG. 15 shows the morphology of motor neuron cells in the upper left, the morphology of dopamine neuron cells in the upper right, the immunofluorescence results of motor neuron precursor cells in the middle, and the immunofluorescence results of dopamine neuron precursor cells at the bottom.
FIG. 16 shows the electrophysiological results of motor neurons cultured for 20 days (A-C) and dopamine neurons cultured for 22 days (D-F), where A shows Na⁺ channel potential in motor neurons, B shows K⁺ channel potential in motor neurons, C shows evoked action potential in motor neurons, D shows Na⁺ channel potential in dopamine neurons, E shows Ka⁺ channel potential in dopamine neurons, and F shows evoked action potential in dopamine neurons.
FIG. 17 shows the electron microscopy image of NSC-EV
FIG. 18 shows the purity of NSC-EV detected by nano-flow cytometry.
FIG. 19 shows the particle size of NSC-EV detected by nano-flow cytometry.
FIG. 20 shows the NSC-EV protein detected by NSC-EV proteomics aligned in the Vesiclepedia database.
FIG. 21 shows the results of GO enrichment analysis of NSC-EV proteomic data.
FIG. 22 shows the morphology of NSCs in the purified supernatant, where A shows the morphology under electron microscopy after tangential flow ultrafiltration, B shows the morphology of fractions 1#-6# and 7#-11# after density gradient centrifugation, and C shows the morphology after purification by QA column.
FIG. 23 shows samples from each purification step detected by nano-flow cytometry, where A shows tangential flow ultrafiltration, B shows fractions 1#-13# of density gradient centrifugation, C shows fraction 7#, and D shows concentration and purity after purification by QA column.
FIG. 24 shows the protein expression of EV markers CD63 and CD81 in refined purified samples as characterized by WB.
FIG. 25 shows that 1×10⁹ p/mL NSC-EV can significantly inhibit the glial cell inflammatory response caused by LPS.
FIG. 26 shows that 1×10⁹ p/mL NSC-EV can significantly inhibit neural apoptosis.

### DETAILED DESCRIPTION

The present disclosure provides human induced pluripotent stem cells overexpressing TLX and use thereof. Those skilled in the art can learn from the contents of the present disclosure and appropriately improve the process parameters. It should be particularly indicated that, all similar replacements and changes are obvious for those skilled in the art, and are deemed to be included in the present disclosure. The method and use of the present disclosure have been described through preferred embodiments, and those skilled apparently can make modifications or appropriate changes and combinations of the method and use herein without departing from the content, spirit and scope of the present disclosure to realize and apply the technology of the present disclosure.

Unless defined otherwise, all technical and scientific terms used herein have the same meaning as understood by those ordinarily skilled in the art. For definitions and terms in the art, professionals can in particular refer to Current Protocols in Molecular Biology (Ausubel). The abbreviations for amino acid residues are the standard 3-letter and/or 1-letter codes used in the art to refer to one of the 20 commonly used L-amino acids.

The full-length TLX protein consists of 385 amino acid residues with a sequence set forth in SEQ ID NO: 1, specifically:

MSKPAGSTSRILDIPCKVCGDRSSGKHYGVYACDGCSGFFKRSIRRNRTYVCKSGNQGGC
PVDKTHRNQCRACRLKKCLEVNMNKDAVQHERGPRTSTIRKQVALYFRGHKEENGAAA
HFPSAALPAPAFFTAVTQLEPHGLELAAVSTTPERQTLVSLAQPTPKYPHEVNGTPMYLYE
VATESVCESAARLLFMSIKWAKSVPAFSTLSLQDQLMLLEDAWRELFVLGIAQWAIPVDA
NTLLAVSGMNGDNTDSQKLNKIISEIQALQEVVARFRQLRLDATEFACLKCIVTFKAVPTH
SGSELRSFRNAAAIAALQDEAQLTLNSYIHTRYPTQPCRFGKLLLLLPALRSISPSTIEEVFF
KKTIGNVPITRLLSDMYKSSDI.

The nucleic acid sequence encoding the TLX protein after codon optimization totals 1155bp with a sequence set forth in SEQ ID NO: 3, specifically:

atgagcaagccagccggatcaacaagccgcattttagatatcccctgcaaagtgtgtggcgaccgcag
ctcggggaagcactacggggtctacgcctgcgacggctgctcaggttttttcaaacggagcatccgaaggaataggacctatgtctgcaaat
ctggaaaccagggaggctgtccggtggacaagacgcacagaaaccagtgcagggcgtgtcggctgaagaagtgtttggaagtcaacatg
aacaaagacgccgtgcagcacgagcgggggcctcggacgtccaccatccgcaagcaagtggccctctacttccgtggacacaaggagg
agaacggggccgccgcgcactttccctcggcggcgctccctgcgccggccttcttcaccgcggtcacgcagctggagccgcacggcctg
gagctggccgcggtgtccaccactccagagcggcagaccctcgtgagcctggctcagcccacgcccaagtacccccatgaagtgaatgg
gaccccaatgtatctctatgaagtggccacggagtcggtgtgtgaatcagctgccagacttctcttcatgagcatcaagtgggctaagagtgt
gccagccttctccacgctgtctttgcaagaccagctgatgcttttggaagatgcttggagagaactgtttgttctaggaatagcacaatgggcc

attccggttgatgctaacactctactggctgtatctggcatgaacggtgacaacacagattcccagaagctgaacaagatcatatctgaaatac
aggctttacaagaggtggtggctcgatttagacaactccggttagatgctactgaatttgcctgtctaaaatgcatcgtcactttcaaagccgttc
ctacacatagtggttctgaactgagaagtttccggaatgctgccgccattgcagcccttcaagatgaggctcagctaacgctcaacagctaca
tccataccagatatcccactcaaccctgtcgctttggaaaactcctgttgcttttgccagctttacgttctattagcccatcaactatagaagaagt
gtttttcaaaaaaaccatcggcaatgtgccaattacaagactgctttcagatatgtacaaatccagtgatatc.

The present disclosure shows that the truncating variant of TLX has a higher expression level in iPSCs compared to the full-length TLX, and the proliferation rate of hiPSCs expressing the truncating variant is higher. However, the proliferation rate of genetically modified hiPSCs was significantly increased compared to that of wild-type hiPSCs, whether expressing the full-length sequence or the truncated variant of TLX.

In the present disclosure, the truncating variant has a deletion of 1-200 amino acids from the N-terminus relative to the TLX amino acid sequence, for example, a deletion of 1-10, 10-20, 20-50, 50-70, 70-100, 100-150, 150-160, 160-170, 170-180, 180-190, or 190-200 amino acids.

In some embodiments, the truncating variant has a deletion of 181 amino acids from the N-terminus relative to the TLX amino acid sequence, and its amino acid sequence is set forth in SEQ ID NO: 2, consisting of a total of 204 amino acid residues, specifically:

TESVCESAARLLFMSIKWAKSVPAFSTLSLQDQLMLLEDAWRELFVLGIAQWAIPVDANT
LLAVSGMNGDNTDSQKLNKIISEIQALQEVVARFRQLRLDATEFACLKCIVTFKAVPTHSG
SELRSFRNAAAIAALQDEAQLTLNSYIHTRYPTQPCRFGKLLLLLPALRSISPSTIEEVFFKK
TIGNVPITRLLSDMYKSSDI.

The nucleic acid sequence encoding the truncating variant of TLX set forth in SEQ ID NO: 2 after codon optimization totals 615bp with a sequence set forth in SEQ ID NO: 4, specifically:

atgacggagtcggtgtgtgaatcagctgccagacttctcttcatgagcatc

aagtgggctaagagtgtgccagccttctccacgctgtctttgcaagaccagctgatgcttttggaagatgcttggagagaactgtttgttctagg

aatagcacaatgggccattccggttgatgctaacactctactggctgtatctggcatgaacggtgacaacacagattcccagaagctgaacaa

gatcatatctgaaatacaggctttacaagaggtggtggctcgatttagacaactccggttagatgctactgaatttgcctgtctaaaatgcatcgt

cactttcaaagccgttcctacacatagtggttctgaactgagaagtttccggaatgctgccgccattgcagcccttcaagatgaggctcagcta

acgctcaacagctacatccataccagatatcccactcaaccctgtcgctttggaaaactcctgttgcttttgccagctttacgttctattagcccat
caactatagaagaagtgtttttcaaaaaaaccatcggcaatgtgccaattacaagactgctttcagatatgtacaaatccagtgatatc.

The present disclosure shows through experiments that the promoter has a significant effect on the expression level of TLX in cells. The expression level of TLX in iPSCs transfected under CMV promoter is significantly higher than that in iPSCs transfected under EF1A promoter.

Preliminary studies have demonstrated that overexpressing TLX in NSCs not only has problems of limited access to NSCs but also involves ethical concerns. More importantly, NSCs overexpressing TLX are unstable in passaging. In the present disclosure, a hiPSC cell line stably overexpressing TLX is obtained by overexpressing TLX in the hiPSC stage, which provides a sufficient number of cell populations to obtain NSC^{TLX}. Additionally, it was found in the experiments that TLX may have a promoting effect on the differentiation of hiPSCs into NSCs. Thus, in an embodiment of the present disclosure, conversion of hiPSC^{TLX} into NSC^{TLX} only takes six days. However, wild-type hiPSCs without overexpressing TLX in the same culture medium fail to be converted to NSCs. Alternatively, adding SMAD small molecule inhibitor (compound C) to NGD medium for 9 days of culture of wild-type hiPSCs is required to obtain cells with low expression of Nestin and Sox2, and 14-21 days of culture is usually required to obtain a high-purity NSC cell population differentiated from wild-type hiPSCs.

The hiPSCs that self-drivingly differentiate into NSCs provided by the present disclosure expresses the nucleic acid of the present disclosure. That is, the hiPSCs express a nucleic acid encoding the protein set forth in SEQ ID NO: 1, or a nucleic acid encoding the truncating variant set forth in SEQ ID NO: 2.

In the present disclosure, hiPSC^{TLX} that can be stably passaged is obtained by infecting hiPSCs with lentivirus. It is also proved that TLX can be used as an internal driving force for the differentiation of hiPSCs into NSCs to drive hiPSCs to differentiate into NSCs, and to obtain NSC^{TLX} which can maintain self-renewal for a long period of time. In the present disclosure, hiPSCs overexpressing TLX under NGDI culture conditions shows a higher expression level of NSC biomarkers than wild-type hiPSCs under conventional differentiation conditions.

In the present disclosure, NSCs differentiated from hiPSCs by culturing hiPSC^{TLX} cells transfected with truncated TLX virus can maintain stable passage and maintain good stemness and differentiation potential.

In the present disclosure, the passaging of NSCs is stably maintained in vitro. The supernatant of NSC culture is collected, and exosomes are extracted in the supernatant of NSCs using existing exosome extraction technology. It is found that the exosomes contain proteins related to biological processes such as immune regulation, signal transduction, and intercellular communication, and also contain a large number of miRNAs related to the nervous system.

It has been experimentally verified that the exosomes of NSCs of the present disclosure have a good protective effect on neurons. The culture supernatant of glial cells stimulated by LPS has a killing effect on nerve cells, but after NSC-EV is added to the culture supernatant, neuronal apoptosis is significantly lower than that of the control group without NSC-EV

The test materials used in the present disclosure are all common commercial products and can be purchased in the market. The present disclosure is further illustrated below in conjunction with the examples.

### Example 1 Plasmid construction and viral packaging for TLX (or called NR2E1)

TLX can maintain NSC proliferation and stemness. Based on the principle that viruses can genetically recombine, a lentiviral plasmid carrying the TLX gene was constructed, and the plasmid was packaged in a lentivirus.

Lentiviral vectors are vectors for gene therapy developed based on HIV-1. Different from general retroviral vectors, they have the ability to infect both dividing and non-dividing cells.

Lentiviral vectors can effectively integrate exogenous genes into host chromosomes to achieve persistent expression of target sequences. In terms of infection ability, it can effectively infect various types of cells such as neuronal cells and stem cells. For some cells that are difficult to be transfected by other means, such as primary cells and stem cells, the use of lentiviral vectors can greatly improve the transduction efficiency of the target genes, and the chances of the target genes being integrated into the host cell genome are greatly increased, so that the long-term, stable expression of the target genes can be achieved more conveniently and quickly.

### 1. Experimental steps

1) Production of target gene fragments: The target gene having nucleic acid sequences set forth in SEQ ID NO: 3-4 was amplified using high-fidelity Prime STAR enzyme, wherein the sequence set forth in SEQ ID NO: 3 encodes the full-length TLX (noted as TLX-FL) set forth in SEQ ID NO: 1, and the sequence set forth in SEQ ID NO: 4 encodes the truncating variant of TLX (noted as TLX-TP) set forth in SEQ ID NO: 2. The resulting products after PCR were subjected to electrophoresis on agarose gel to detect the amplification effect. The target gene band were cut off from the gel after electrophoresis, and recovered using a gel DNA recovery kit. In addition, the target gene was ligated with the gene encoding green fluorescent protein (EGFP) to obtain a fusion fragment.
2) Preparation of linearized expression vector: The expression vector was digested with restriction endonuclease with the following reaction system: 2 µg of plasmid, 5 µL of 10× reaction buffer, 1 µL of each of the restriction endonuclease, and water up to 50 µL, and incubated in water bath at 37°C for more than 2 h. After digestion, the resulting products were detected by agarose gel electrophoresis, and the target vector band was cut off from the gel after agarose gel electrophoresis and recovered using a gel DNA recovery kit.
3) Introduction of target gene into linearized expression vector: Using the seamless assembly kit, the target gene fragment and linearized vector were added to a centrifuge tube at a molar ratio of 1:1, mixed, incubated at 37°C for 30 min, and then placed on ice for 5 min.
4) Competent cell transformation: The constructed expression vector was transformed into E. coli competent cells.
5) Identification of positive transformants by colony PCR: The transformants grown on the plate were picked and resuspended in 10 µL of LB culture medium, and 1 µL was used as a template for identification by colony PCR.
6) The positive clones were subjected to sequencing.
7) Plasmids were extracted using a plasmid miniprep kit.
8) Viral packaging: 4×10⁶ cells were seeded into a 100-mm cell culture dish at a density of 70-80%. One day later, the viral vector plasmid and transfection reagents were added to this cell culture dish. After 6h, the medium was replaced with fresh complete medium. The virus was collected at 48h and 72h separately for purification.
9) Virus verification: 293T cells were infected with the obtained virus and the expression of the target gene was detected by WB.

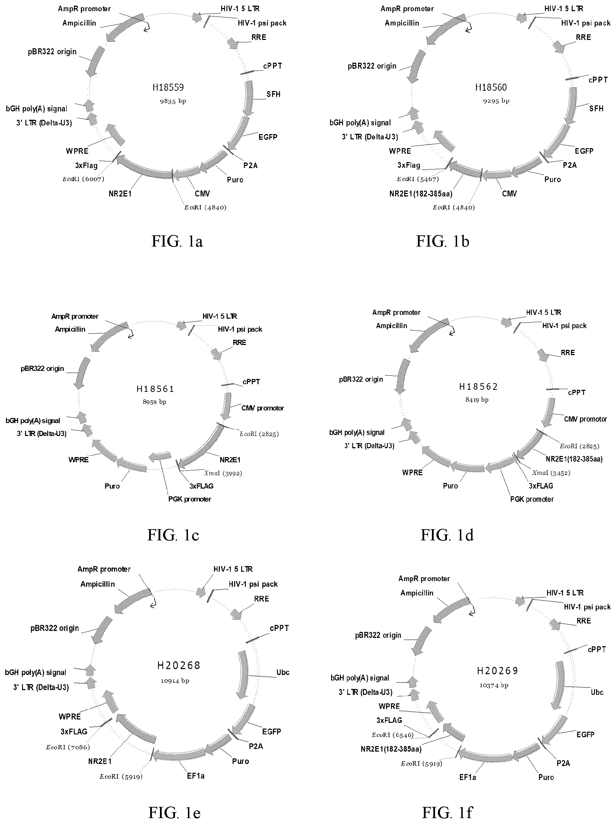

The experimental results are shown in FIGs. 1-2.

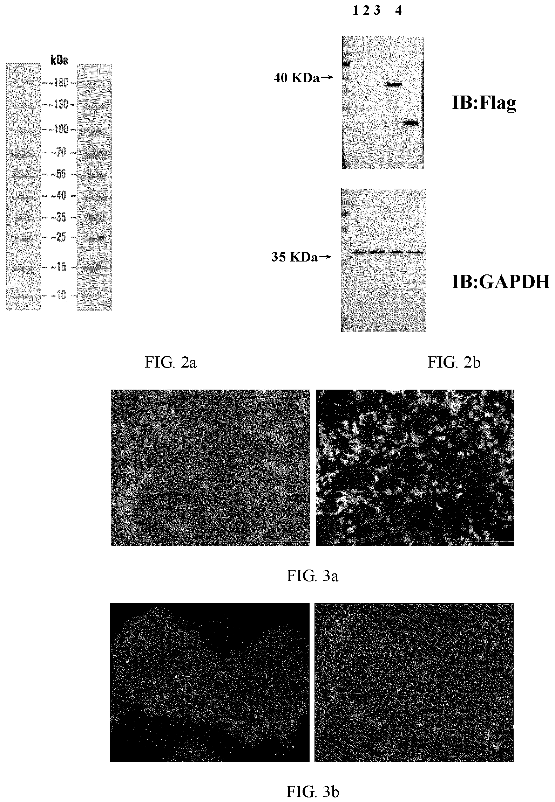

Experimental conclusion: The plasmids were successfully constructed using the above method (The plasmid maps are shown in FIGs. 1a-1f), and the virus was able to infect cells, allowing cells to express the target gene (FIGs. 2a and 2b).

### Example 2 Infection of hiPSCs with TLX lentivirus

hiPSCs were infected with lentivirus to overexpress TLX in hiPSCs, and a stably passaged hiPSC^{TLX} cell line was obtained. The experimental steps are as follows.
1) Cells were seeded into a 12-well plate at 2.5×10⁵ cells/well and incubated for 24 h.
2) The culture medium was replaced with fresh medium containing virus at MOI=10. The cells transfected with lenti-SFH-EGFP-CMV-NR2E1 were noted as hiPSC^{FL-TLX}; and cells transfected with lenti-SFH-EGFP-CMV-NR2E1 (182-386aa) were noted as hiPSC^{TP-TLX}.
3) After 16 h of cell culture, the medium was removed and replaced with fresh medium daily, and the cells were passaged every 3 days.

### Experimental results and conclusions

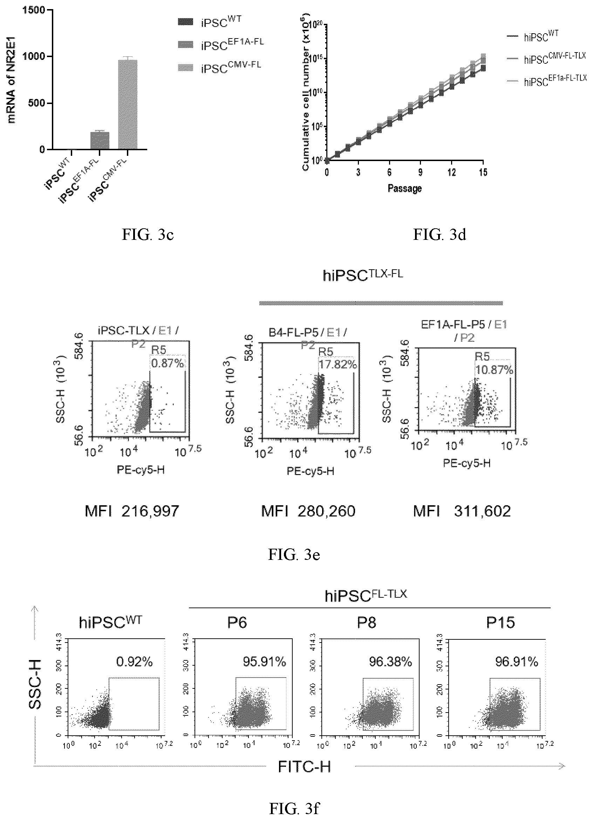

FIG. 3a and FIG. 3b show that the cells were successfully infected with TLX virus, and that overexpression of full-length TLX had no effect on the morphology of iPSCs. FIG. 3c shows that infected hiPSCs were found to highly express the full-length TLX gene as detected by qPCR, and between the two promoters, the CMV promoter was more favorable for the expression of full-length TLX. FIG. 3d shows that the expression of full-length TLX had no effect on the iPSC growth regardless of which promoter was used for overexpression. In addition, both of the two promoters can allow the expression of full-length TLX in hiPSCs (FIG. 3e). Moreover, expression of EGFP had no effect on cell growth and TLX function (FIG. 3f).

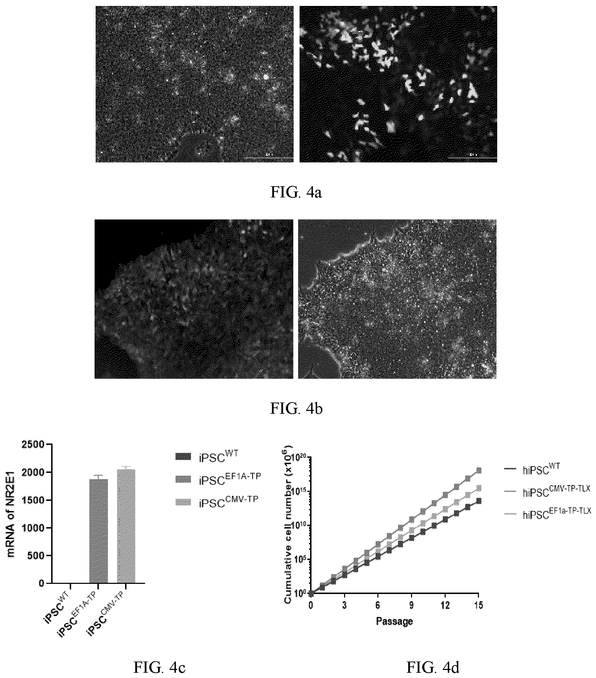

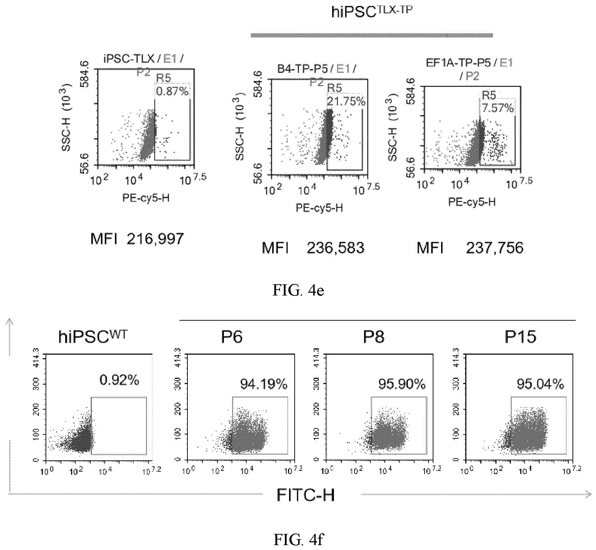

FIG. 4a and FIG. 4b show that the cells were successfully infected with TLX virus, and that overexpression of the truncated TLX had no effect on the morphology of iPSCs. FIG. 4c shows that infected hiPSCs were found to highly express the truncated TLX gene as detected by qPCR, and between the two promoters, the CMV promoter was more favorable for the expression of truncated TLX. FIG. 4d shows that the expression of truncated TLX had no effect on the iPSC growth regardless of which promoter was used for overexpression. In addition, both of the two promoters can allow the expression of truncated TLX in hiPSCs (FIG. 4e). Moreover, it can be seen from FIG. 4f that expression of EGFP had no effect on cell growth and TLX function.

### Example 3 Self-driven differentiation of hiPSC^{TLX} into NSCs

Experimental purpose: Achieving differentiation of iPSCs into NSCs by using exogenous TLX or TLX truncating variant overexpressed in iPSCs to drive iPSCs under NSC medium conditions. The untransfected wild-type iPSCs were used as a contro. The iPSCs used expressed EGFP and overexpressed TLX or truncated TLX. EGFP fluorescent protein was used as a reporter gene to observe the culture in the medium. The cells expressing TLX or truncated TLX but not EGFP were also cultured to prove that the expression of EGFP had no effect on the culture.

### Experimental steps

According to cells and media, the experiments include the following groups:
Control: Differentiation of hiPSCs without TLX overexpression): Cells were induced for differentiation with SB431542, LDN193189 and LIF from day 0 to 7, further induced until day 14 with SB431542, and further induced until day 21 with FGF2 and EGF.

Group a (NGD): The culture medium consisted of Neurobasal medium, 2 g/100 mL BSA, 1 vol% Glutamax additive, 1 vol% sodium pyruvate, 0.05 mol/L NaCl, 5 vol% N2 trophic factor, 10 vol% B27 neurotrophic factor, and 1×penicillin-streptomycin. Cells were seeded into a 6-well plate at 2.5×10⁵ cells/well using Epic medium. The next day, the medium was replaced with the medium in Group a. After 3 days of culture, the cells did not show the typical morphology Rosette (flower clusters) of NSCs.

Group b (NGD-F): 10 ng/mL FGF was added to the NGD medium. Cells were seeded at 2.5×10⁵ cells/well into a 6-well plate using Epic medium. The next day, the medium was replaced with the medium in Group a. After 3 days of culture, rosette morphology was observed in NSCs.

Group c (NGD-C): 2.5 µM compound C was added to the NGD medium. Cells were seeded at 2.5×10⁵cells/well into a 6-well plate using Epic medium. The next day, the medium was replaced with the medium in Group a. After 3 days of culture, rosette morphology was not observed in cells.

Group d (NGD-L): 10 ng/mL LIF was added to the NGD medium. Cells were seeded at 2.5×10⁵cells/well into a 6-well plate using Epic medium. The next day, the medium was replaced with the medium in Group a. After 3 days of culture, rosette morphology was not observed in cells.

Group e (NGD-FI): 10 ng/mL FGF2 and 10 ng/mL insulin were added to the NGD medium. Cells were seeded at 2.5×10⁵cells/well into a 6-well plate using Epic medium. The next day, the medium was replaced with the medium in Group a. After 3 days of culture, rosette morphology was not observed in cells.

Group f (NGD-I): 10 ng/mL FGF2 and 10 ng/mL insulin were added to the NGD medium. Cells were seeded at 2.5×10⁵cells/well into a 6-well plate using Epic medium. The next day, the medium was replaced with the medium in Group a. After 3 days of culture, rosette morphology was not observed in cells.

Group g (NGD-FIL): 10 ng/mL FGF2, 10 ng/mL insulin and 10 ng/mL LIF were added to the NGD medium. Cells were seeded at 2.5×10⁵cells/well into a 6-well plate using Epic medium. The next day, the medium was replaced with the medium in Group a. After 3 days of culture, rosette morphology was not observed in cells.

Group h (LSL): 10 µM SB431542, 0.25 µM LDN193189 and 10 ng/mL LIF were added to the ncEpic medium. Cells were seeded at 2.5×10⁵cells/well into a 6-well plate using Epic medium. The next day, the medium was replaced with the medium in Group a. After 3 days of culture, the cells were dead.

Group i (LSDL): 10 µM SB431542, 0.25 µM LDN193189, 2.5 µM DMH-1 and 10 ng/mL LIF were added to the ncEpic medium. Cells were seeded at 2.5×10⁵ cells/well into a 6-well plate using Epic medium. The next day, the medium was replaced with the medium in Group a. After 3 days of culture, the cells were dead.

Group j (NIM): 0.5 mL of N2, 0.5 mL of Glutamax, 0.5 mL of NEAA and 10 ng/mL Heparin were added to the DMEM/F12 medium. Cells were seeded at 2.5×10⁵cells/well into a 6-well plate using Epic medium. The next day, the medium was replaced with the medium in Group a. After 3 days of culture, rosette morphology was not observed in cells.

Group k (NIM++): 0.5 mL of N2, 0.5 mL of Glutamax, 0.5 mL of NEAA, 10 ng/mL Heparin, 10 ng/mL FGF2 and 10 ng/mL EGF were added to the DMEM/F12 medium. Cells were seeded at 2.5×10⁵cells/well into a 6-well plate using Epic medium. The next day, the medium was replaced with the medium in Group a. After 3 days of culture, rosette morphology was not observed in cells.

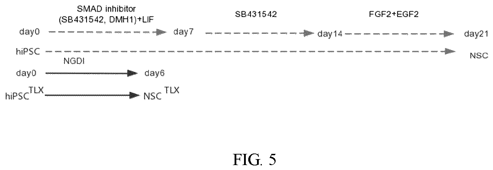

### Experimental results and conclusions (FIGs. 5-6)

As shown in the flow chart for differentiation in FIG. 5, taking NGDI medium as an example, the differentiation time was significantly shortened, and the differentiation process and the reagents used was significantly simplified in the present disclosure compared to the conventional method for differentiating hiPSCs into NSCs.

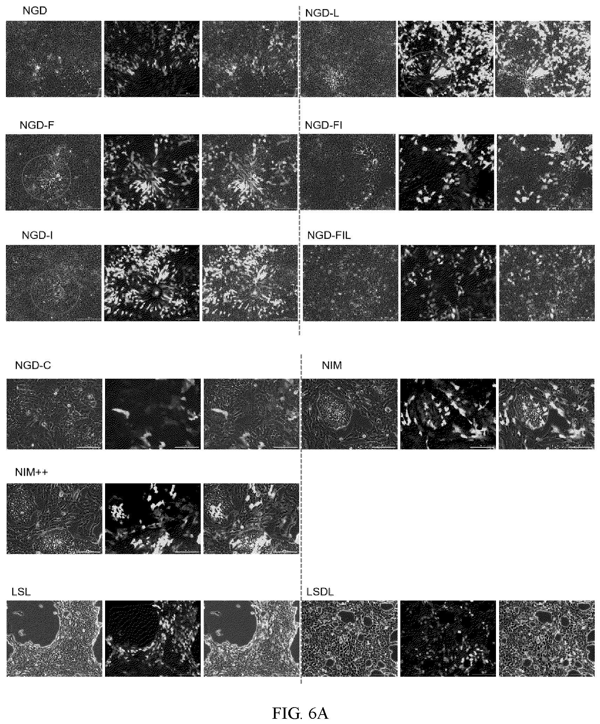

After inducing differentiation of iPSC^{TLX-FL} into iNSC^{TLX-FL} with different differentiation media, only cells cultured in NGD-F, NGD-I, NGD-L media showed rosette morphology. After several replicates, NGD-I medium showed the best replication of the experimental results. It was clearly shown that cells cultured in conventional differentiation media for NSCs (SLS and LSDL media) died in large numbers, with almost all cells shedding and dying after 3 days of culture (FIG. 6A).

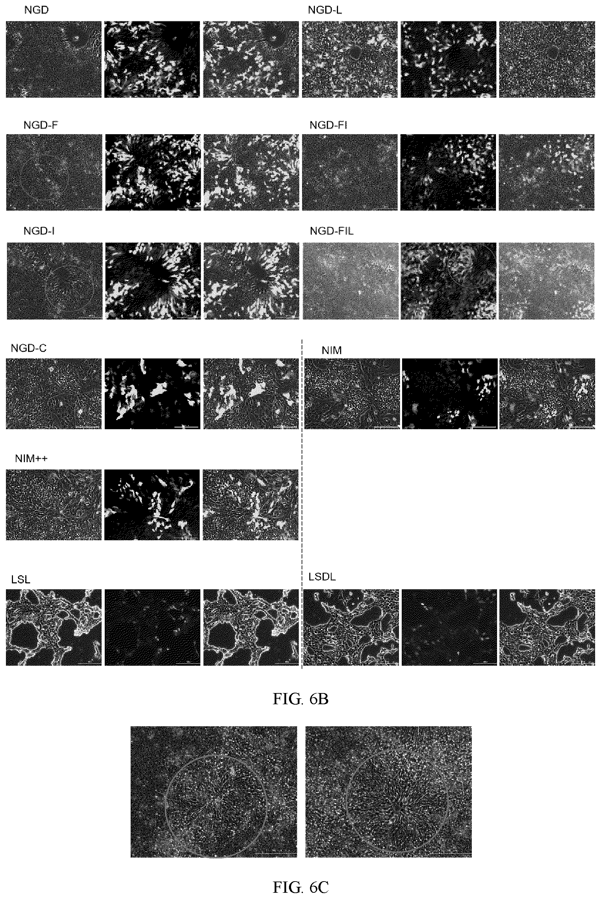

After inducing differentiation of iPSC^{TLX-TP} into iNSC^{TLX-TP} with different differentiation media, only cells cultured in NGD-F and NGD-I media showed rosette morphology. After several replicates, NGD-I medium showed the best replication of the experimental results. It was clearly shown that cells cultured in conventional differentiation media for NSCs (SLS and LSDL media) died in large numbers, with almost all cells shedding and dying after 3 days of culture (FIG. 6B).

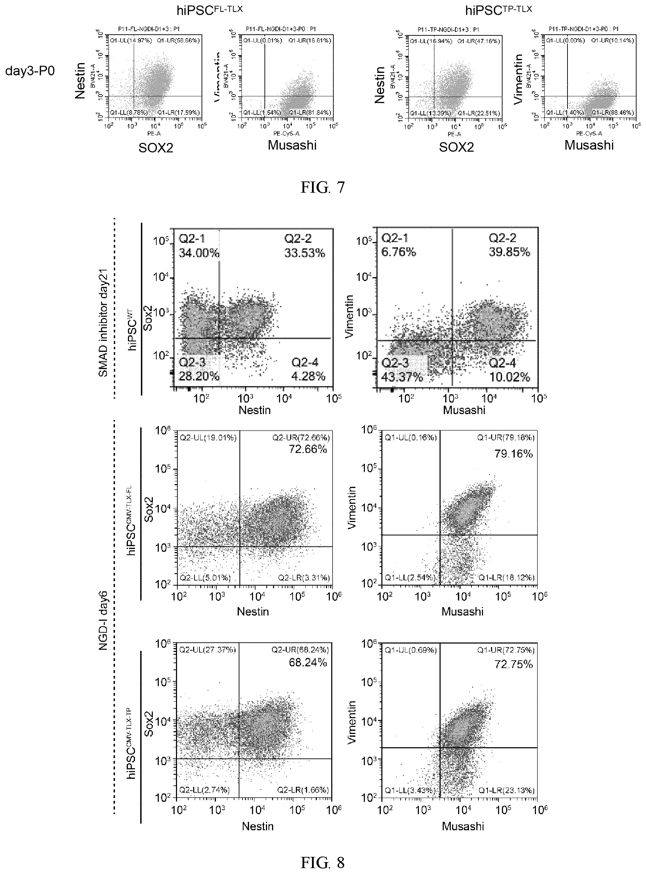

In addition, hiPSCs overexpressing TLX without EGFP gene showed rosette morphology of NSCs after 3 days of culture in NGD-I medium (FIG. 6C). The cells with such morphology expressed NSC marker proteins (Nestin, Sox2, Vimentin, Musashi) as detected by flow cytometry (FIG. 7).

As shown in FIG. 8, hiPSCs overexpressing TLX were cultured in NGDI medium for 6 days, and the double-positive proportion of Nestin⁺/Sox2⁺ and Vimentin+/Musashi+ in the cells was significantly higher than that in wild-type hiPSCs differentiated for 21 days using conventional differentiation methods, indicating that the method of the present disclosure can obtain NSCs with a higher positive rate in a very short experimental period than conventional methods.

### Example 4 Stable subculture of NSCs

In order to achieve long-term stable subculture of NSC^{TLX}, cells of NSC^{TLX-FL} and NSC^{TLX-TP} obtained by differentiation in Example 3 or cells obtained by differentiation in the control group were passaged at 0.25×10⁵ cells/cm² every 5 days (Grouping was performed according to the selected medium). Cells used for subculture did not express EGFP, but expressed TLX or truncated TLX. Matrigel, Vitronectin or Laminin was used to coat the wells of the plate.

Group 1 (NGD-I): The culture medium consisted of Neurobasal medium, 2g/100mL BSA, 1 vol% Glutamax additive, 1 vol% sodium pyruvate, 0.05 mol/L NaCl, 5 vol% N2 trophic factor, 10 vol% B27 neurotrophic factor and 1×penicillin-streptomycin, to which 10 ng/mL insulin was added.

Group 2 (NIM): 0.5 mL N2, 0.5 mL Glutamax, 0.5 mL NEAA, and 10 ng/mL Heparin were added to DMEM/F12 medium.

Group 3 (NIM++): 0.5 mL N2, 0.5 mL Glutamax, 0.5 mL NEAA, 10 ng/mL Heparin, 10 ng/mL FGF2 and 10 ng/mL EGF were added to DMEM/F12 medium. Cells can be stably passaged.

Group 4 (TFM): DMEM/F12 medium and Neurobasal medium were mixed at an ratio of 1:1, and 2% TFM additive (containing GSK3 and TGF-β inhibitors) was added. Cells can be stably passaged for a long term.

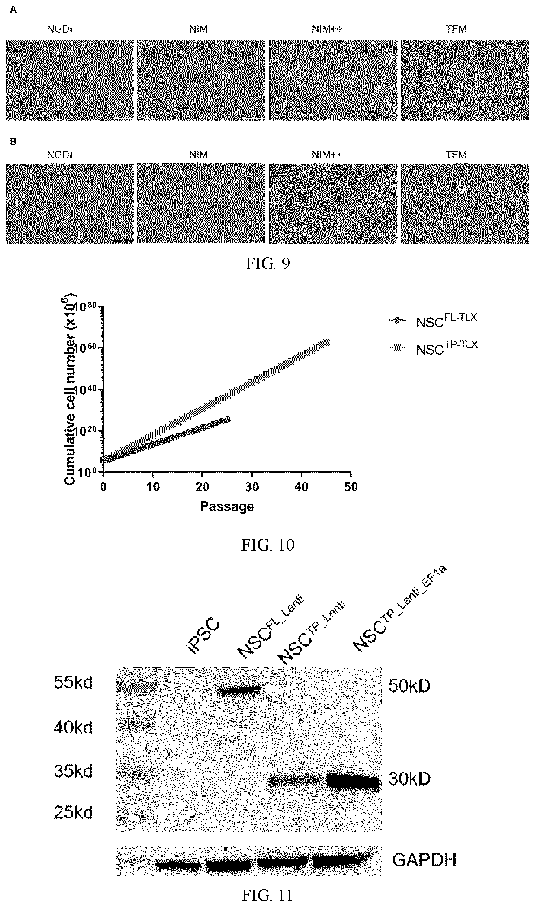

The results are shown in FIG. 9 that iNSC^{TLX} can be passaged well in all four maintenance media. The medium containing GSK3 and TGF-beta pathway inhibitors (in this example, taking commercially available culture medium containing GSK3 and TGF-β inhibitors as an example, i.e., TFM media) can better maintain the stability of iNSC^{TLX} in passage.

As shown in Figure 10, the growth rate of iNSC^{TLX} was monitored, and it was found that all iNSC^{TLX} stably proliferated and passaged at a high rate. In addition, the growth rate of iNSC^{TLX-TP} was significantly higher than that of iNSC^{TLX-FL}.

As shown in Figure 11, Western Blot was used to detect Flag tags in NSCs. NSC^{FL-lenti} and NSC^{TP-lenti} indicated the cells transfected with viruses lenti-Ub-EGFP-EF1a-NR2E1 and lenti-Ub-EGFP-EF1a-NR2E1 (182-386aa), respectively, and NSC^{TP-lenti-EF1a} represents the NSC^{TP-lenti} cells further transfected with the virus lenti-Ub-EGFP-EF1a-NR2E1 (182-386aa). Flag tags were expressed in fusion with NR2E1. It was indicated that TLX transfected into hiPSCs was still stably expressed in NSCs by detecting the expression of Flag tags.

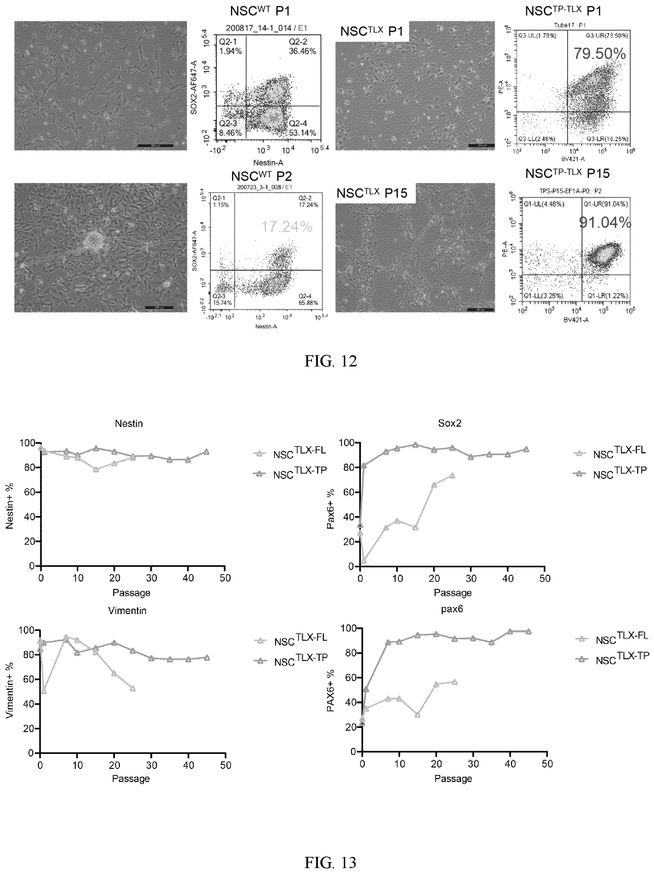

FIG. 12 shows the morphology of NSCs differentiated from wild-type hiPSCs at passage 1 (P1) and 2 (P2) and the results of expression levels of SOX2/Nestin by flow cytometry; and the morphology of NSCs differentiated from iNSC^{TLX-TP} at passage 1 (P1) and 15 (P15) and the results of expression levels of SOX2/Nestin by flow cytometry. When wild-type iNSCs were passaged at P2, neuronal differentiation can be clearly seen, and expression of NSC protein markers was decreased. In additioin, FIG. 13 shows the expression levels of SOX2, Nestin, Vimentin, and PAX6 in iNSC^{TLX} cells at different passages detected by flow cytometry. iNSC^{TLX-TP} cells at passage 45 can still stably express SOX2, Nestin, Vimentin, and PAX6 proteins, indicating that the present disclosure achieves a breakthrough in NSC passage from 1 to at least 45 passages.

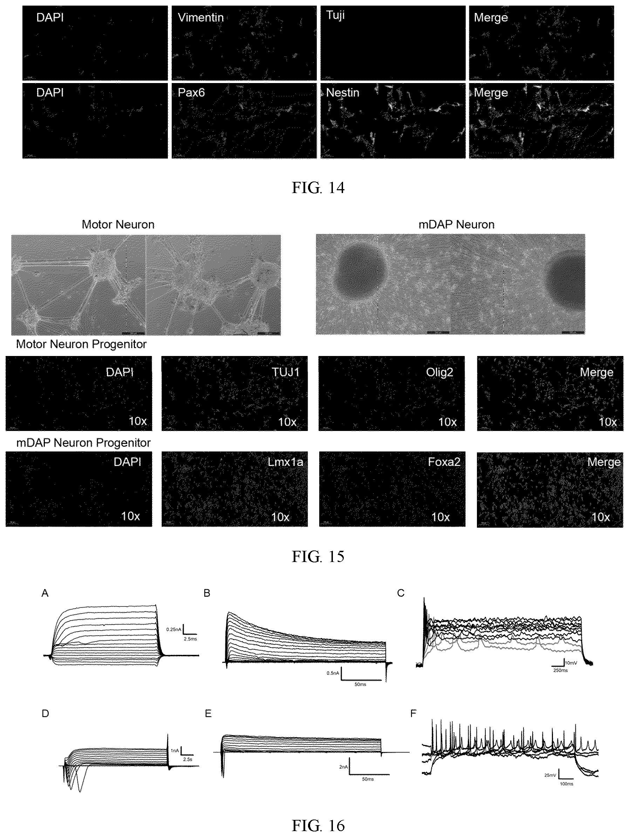

In addition, the expression of Vimentin, Tuj1, Pax6, and Nestin in iNSC^{TLX-TP} cells detected by immunofluorescence assay again demonstrated that iNSC^{TLX-TP} cells highly expressed NSC biomarkers (Vimentin, Pax6, Nestin), but did not express neuronal markers (Tuj1) (FIG. 14).

### Example 5 Characterization of NSC stemness maintenance

In order to prove that overexpression of TLX does not affect the stemness of NSCs, dopamine neuron and motor neuron differentiation kits were used to differentiate NSCs into dopamine neurons and motor neurons, respectively.

FIG. 15 shows the morphology of motor neuron cells in the upper left, the morphology of dopamine neuron cells in the upper right, the immunofluorescence results of motor neuron precursor cells in the middle, and the immunofluorescence results of dopamine neuron precursor cells at the bottom. It could be seen from the immunofluorescence results that NSC can successfully differentiate into dopamine and motor neuron precursor cells, indicating that overexpression of TLX had no effect on the stemness of NSCs.

FIG. 16 shows the electrophysiological results of motor neurons cultured for 20 days (A-C) and dopamine neurons cultured for 22 days (D-F), where A shows Na⁺ channel potential in motor neurons, B shows K⁺ channel potential in motor neurons, C shows evoked action potential in motor neurons, D shows Na⁺ channel potential in dopamine neurons, E shows Ka⁺ channel potential in dopamine neurons, and F shows evoked action potential in dopamine neurons. It shows that motor neurons and dopamine neurons had matured.

### Example 6 Extraction and characterization of NSC-derived exosomes

The passaging of NSCs was stably maintained in vitro. The supernatant of NSCs was collected, and exosomes were extracted in the supernatant of NSCs using existing exosome extraction technology.

Experimental method: Primary purification of the supernatant of NSCs was performed by tangential flow ultrafiltration, followed by refined purification of the primary purified sample using a Core700 column.

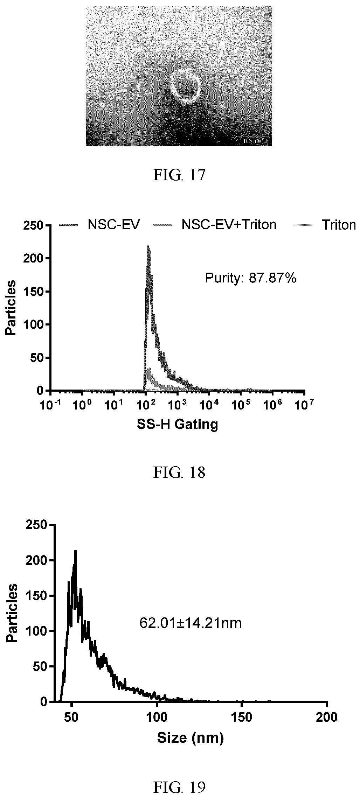

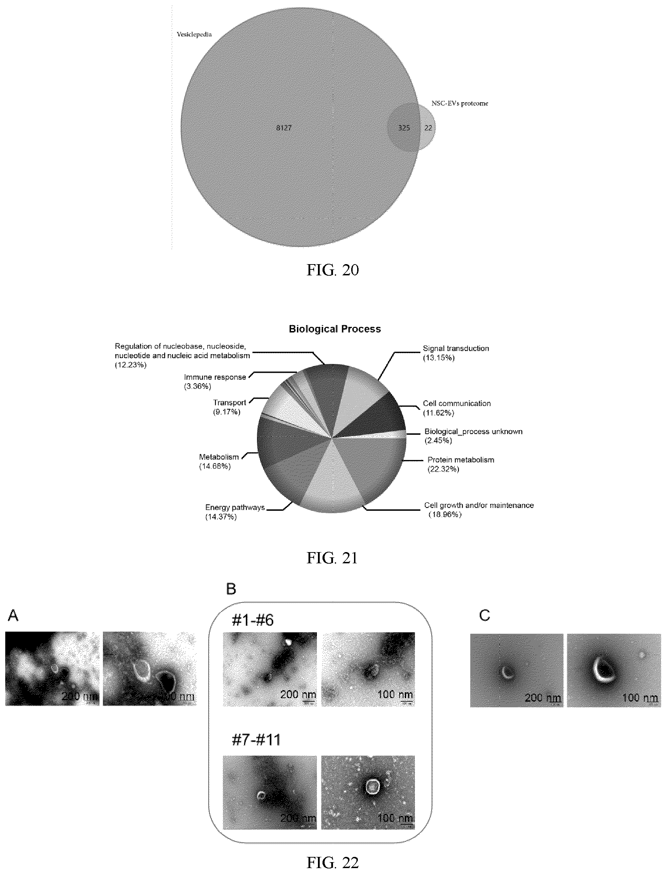

Experimental results and conclusions: FIG. 17 shows the electron microscopy image of NSC-EV, indicating that the NSC-EV obtained by culture had good morphology. The purity of NSC-EV was up to 87.87% (FIG. 18) and the particle size of NSC-EV was 62.01±14.21nm (FIG. 19), as detected by nano-flow cytometry. After aligning the NSC-EV protein detected by NSC-EV proteomics in the Vesiclepedia database, it was found that up to 93.66% of the protein detected by NSC-EV proteomics belonged to extracellular vesicles, indicating the reliability of the purification method (FIG. 20). By performing Go enrichment analysis of the proteomic data of NSC-EV, the biological process was analyzed separately (FIG. 21).

Conclusion: It can be seen from the result that the proteins of NSC-EV contain proteins related to biological processes such as immune regulation, signal transduction, and cell communication.

**Table 1 A large number of miRNAs related to the nervous system in NSC-EV as detected by transcriptomic analysis of NSC-EV**

| Pathway | p value | #genes | miRNA | |
|---|---|---|---|---|
| | | | Number | Names |
| Fatty acid biosynthesis | 4.09E-10 | 4 | 1 | miR-16-5p |
| Signaling pathways regulating pluripotency of stem cells | 2.11E-06 | 76 | 6 | miR-1246, miR-92a-1-5p, miR-92a-1-5p, miR-138-2-3p, miR-3613-3p, miR-1290 |
| Hippo signaling pathway | 0.000264924 | 79 | 4 | miR-16-5p, miR-3613-3p, miR-4655-5p, miR-651-3p |
| ECM-receptor interaction | 0.001077797 | 25 | 7 | miR-6513-5p, miR-92a-1-5p, miR-6756-3p, miR-510-3p, miR-651-3p, miR-6516-5p, miR-6074 |
| Glycosphingolipid biosynthesis - lacto and neolacto series | <1e-325 | 14 | 7 | miR-1246,miR-6513-5p, miR-764,miR-3972, miR-3972,miR-6516-5p, miR-6516-5p |

### Example 7 Purification and characterization of NSC-EV

The passaging of NSCs was stably maintained in vitro. The supernatant of NSCs was collected, and exosomes were extracted in the supernatant of NSCs using existing exosome extraction technology.

Experimental method: Primary purification of the supernatant of NSCs was performed by tangential flow ultrafiltration, followed by further purification by density gradient centrifugation, and refined purification of the primary purified sample using a Core700 column.

### Experimental results:

FIG. 22 shows the morphology under electron microscopy after tangential flow ultrafiltration (A), the morphology of fractions 1#-6# and 7#-11# after density gradient centrifugation (B), and the morphology after purification by QA column (C), indicating that after purification step by step, impurity proteins in the sample were gradually removed.

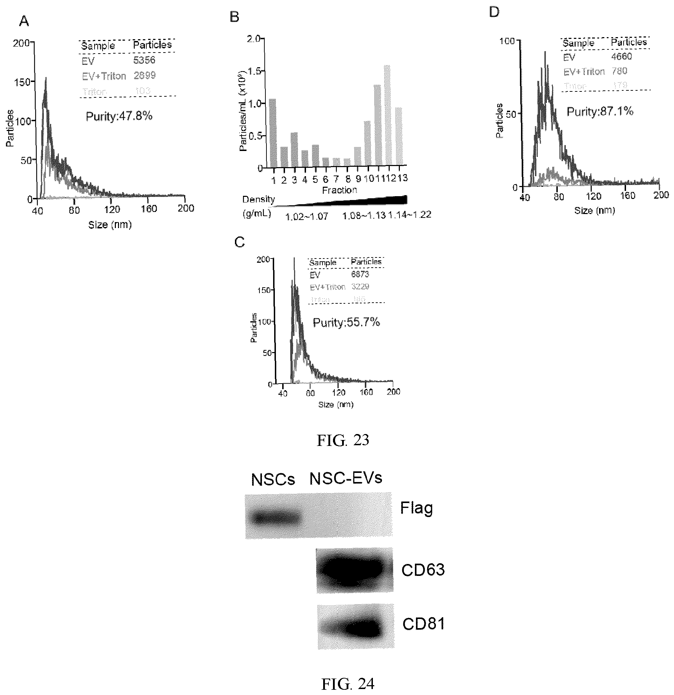

FIG. 23 shows samples from each purification step detected by nano-flow cytometry: tangential flow ultrafiltration (A), fraction 7# (C), concentration and purity after QA column purification (D), and concentration of fractions 1#-13# of density gradient centrifugation, indicating that after purification step by step, the purity of the sample gradually increased.

FIG. 24 shows the protein expression of EV markers CD63 and CD81 in refined purified samples as characterized by WB, and the expression of Flag in NSC-EV characterized using NSCs as a control, indicating that the samples obtained were EVs with high purity and did not carry overexpressed TLX proteins.

### Example 8 Evaluation of pharmacodynamics of NSC-EV

According to the results of proteomic analysis of NSC-EV, NSC-EV contained miRNAs related to the nervous system and proteins related to immunity. The protective effect of NSC-EV on neurons was verified by constructing extracellular nervous system inflammation model.

Experimental methods: Rat primary glial cells were cultured with LPS and different concentrations of NSC-EV for 24 h, and collected for detection of inflammatory factor genes by qPCR. Rat primary glial cells (cultured in the chamber) were cultured with rat primary neuronal cells (cultured in a 6-well plate) by using the co-culture chamber. After 72h, the apoptosis of neuronal cells was detected by flow cytometry.

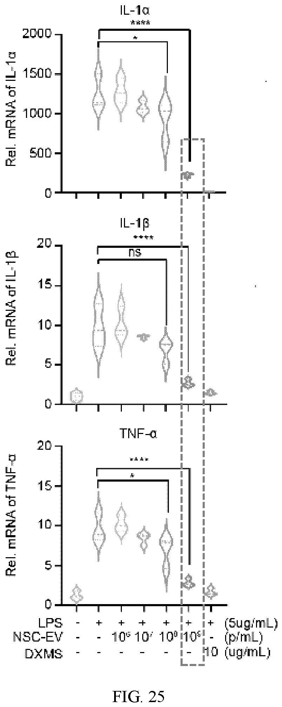

The experimental results are shown in FIG. 25 that the expression levels of inflammatory genes IL-1α, IL-1β and TNF-α in the cells of the group added with 1×10⁹ p/mL NSC-EV were significantly decreased compared to those of the control group (LPS group), indicating that the 1×10⁹ p/mL NSC-EV can significantly inhibit the inflammatory response of glial cells induced by LPS.

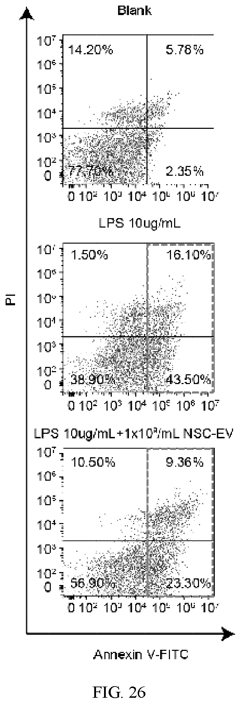

The experimental results are shown in FIG. 26 that the apoptotic cells of neurons cultured in the supernatant of glial cells cultured with 1×10⁹ p/mL NSC-EV and LPS were significantly lower than those in the LPS group, indicating that NSC-EV has a protective effect on neurons.

Preferred embodiments of the present disclosure are described above. It should be noted that those skilled in the art can make several improvements and modifications without departing from the principle of the present disclosure. These improvements and modifications fall within the protection scope of the present disclosure.

## Claims

1. Use of TLX in the construction of human induced pluripotent stem cells (hiPSCs) that self-drivingly differentiate into neural stem cells (NSCs), wherein the TLX comprises at least one selected from the group consisting of the following I to IV:
I), a TLX protein having an amino acid sequence set forth in SEQ ID NO: 1,
II), a protein having an amino acid sequence of the TLX protein in I) by substitution, deletion or addition of one or more amino acids and with a function identical or similar to the TLX protein,
III), a nucleic acid molecule encoding the protein in I) or II), and
IV), a nucleic acid molecule having a nucleotide sequence of the nucleic acid molecule in III) by substitution, deletion or addition of one or more nucleotides and encoding a protein with an identical or similar function; and
V), an substance regulating a level or activity of at least one selected from the group consisting of I) to V).

2. A protein, wherein the protein is
a TLX protein having an amino acid sequence set forth in SEQ ID NO: 1, or
a truncating variant having an amino acid sequence set forth in SEQ ID NO: 1 by deletion of 1-200 amino acids at N-terminus.

3. The protein according to claim 2, wherein the protein is a truncating variant of a TLX protein having an amino acid sequence set forth in SEQ ID NO: 2.

4. A nucleic acid, encoding the protein according to claim 2 or 3.

5. The nucleic acid according to claim 4, wherein
the nucleic acid has an nucleic acid sequence set forth in SEQ ID NO: 3 and encodes a protein set forth in SEQ ID NO: 1, and/or
the nucleic acid has an nucleic acid sequence set forth in SEQ ID NO: 4 and encodes a protein set forth in SEQ ID NO: 2.

6. A transcription unit, comprising a promoter and the nucleic acid according to claim 4 or 5.

7. The transcription unit according to claim 6, wherein the promoter is a CMV promoter or an EF1A promoter.

8. A plasmid vector, comprising a backbone vector and the transcription unit according to claim 6 or 7.

9. A host cell, transfected or transformed with the plasmid vector according to claim 8.

10. A human induced pluripotent stem cell (hiPSC) that self-drivingly differentiates into a neural stem cell (NSC), expressing the nucleic acid according to claim 4 or 5.

11. A method for constructing the hiPSC according to claim 10, comprising
packaging the plasmid vector according to claim 8 and a helper plasmid in a virus to obtain a lentivirus, and
infecting hiPSCs to obtain the hiPSCs that self-drivingly differentiate into NSCs.

12. An immortalized neural stem cell (NSC), obtained by differentiation of the hiPSC according to claim 10.

13. A culture medium, comprising a basal culture medium and one selected from the group consisting of BSA, Glutamax additive, sodium pyruvate, NaCl, N2 trophic factor, B27 neurotrophic factor, insulin, nonessential amino acid, FGF2, EGF, heparin and a combination thereof.

14. A method for differentiating human induced pluripotent stem cells (hiPSCs) into immortalized neural stem cells (NSCs), comprising culturing the hiPSC according to claim 10 to obtain an immortalized NSC.

15. The method according to claim 14, wherein the culturing is carried out with the culture medium according to claim 13.

16. A method for subculturing the immortalized NSC according to claim 12, comprising subculturing using a culture medium comprising GSK3 and TGF-beta inhibitors.

17. A method for producing an exosome of neural stem cells (NSCs), comprising culturing the immortalized NSC according to claim 12, collecting a supernatant, and extracting an exosome of NSCs.

18. An exosome of neural stem cells (NSCs), produced by the method according to claim 17.

19. Use of the exosome of neural stem cells (NSCs) produced by the method according to claim 17 in the manufacture of a medicament for protecting a neuron.

20. A medicament for use in protecting a neuron, comprising the exosome of neural stem cells (NSCs) produced by the method according to claim 17.

21. A method for protecting a neuron, comprising administering the medicament according to claim 20.
